# EUROPEAN PATENT APPLICATION

(11) **EP 4 342 459 A1**
(43) Date of publication of application: **27.03.2024**
(21) Application number: 23382952.2
(22) Date of filing: 20.09.2023
(51) Int. Cl.: A61K 9/20, A61K 31/7048

(54) **PHARMACEUTICAL COMPOSITIONS OF EMPAGLIFLOZIN**

(30) Priority: 20.09.2022 EP 22382868
(71) Applicant: Galenicum Health S.L.U., 08950 Esplugues de Llobregat (ES)
(72) Inventor: GÓMEZ COELLO, Luis, San Agustín de Guadalix (ES); PÉREZ PÉREZ, María del Rocío, San Agustín de Guadalix (ES)
(74) Representative: Galenicum Health S.L.U.

(57) **Abstract**

The present invention relates to stable pharmaceutical compositions of empagliflozin, in the form of immediate release tablets and to a process for the manufacture of said stable pharmaceutical compositions.

## Description

The present invention relates to immediate release pharmaceutical compositions of empagliflozin and to a process for the manufacture of said pharmaceutical compositions.

### STATE OF THE ART

Empagliflozin is a drug used in the treatment of type 2 diabetes. It acts in the kidneys where it inhibits sodium glucose co-transporter-2 (SGLT2). Under normal conditions, SGLT2 is responsible for renal glucose reabsorption from the urine back into the blood, transporting approximately 180 g per day. Consequently, its inhibition allows some glucose to remain in the urine which is then excreted. In this way, empagliflozin lowers blood glucose levels.

Empagliflozin exists in various solid forms. One known crystal form is characterised by having an X-ray powder diffraction (XRPD) pattern that comprises peaks at 14.69, 18.84, 19.16, 19.50, 20.36 and 25.21 degrees 2Θ (±0.05° 2Θ) obtained using CuKα radiation, as described in WO2006117359.

Several pharmaceutical dosage forms of empagliflozin have been published. The main problem encountered when preparing formulations of empagliflozin is low solubility of empagliflozin, leading to difficulties with disintegration and dissolution times. US2017247356 relates to the problem of the low solubility of crystalline empagliflozin and discloses a co-crystal of empagliflozin and an amino acid.

Some of the formulations so far disclosed use wet granulation as the process for the manufacture of empagliflozin formulations. However, the low solubility of empagliflozin in water may require the use of organic solvents, some of which are concerning both for the workers' health and for the environment.

Other approaches have included the use of amorphous empagliflozin. However, to date, these approaches have had the disadvantage that the amorphous forms are usually less stable. Furthermore, the processes used to date to form the amorphous empagliflozin are low-yielding.

Other attempts to address the insolubility of empagliflozin have resulted in manufacturers using empagliflozin with very small particles.

WO2010092126 (WO'126) discloses pharmaceutical compositions comprising empagliflozin as active ingredient, wherein empagliflozin represents 25% or less of the weight of the composition, and wherein the PSD of empagliflozin is D90 ≥ 1 *µ* m and D90 < 200 *µ* m.

A family member of WO'126, EP2395968 disclaims a PSD D90 <10µm, and shows that the working embodiments are between PSD D90 ≥ 22 *µ* m and D90 ≤ 73 *µ* m.

Therefore, there is an unmet medical need for pharmaceutical compositions with a good efficacy, manufactured by safe, effective, easy and environmentally friendly manufacturing methods having a micronized grade PSD.

There is also a need to provide patients with a formulation that affords acceptable-sized tablets or capsules across all of the approved dosages.

### SUMMARY OF THE INVENTION

In a first aspect, the present invention relates to a pharmaceutical composition comprising at least one active ingredient, wherein said active ingredient is the compound of the formula (1) 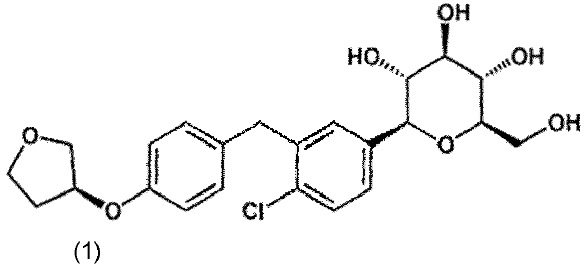 wherein said composition comprises:
at least one diluent,
at least one disintegrant,
at least one lubricant,
wherein the compound of formula (1) has a particle size distribution D90 between 1 and 10 µm,
wherein the composition is prepared preferably by dry methods, and
wherein the composition is preferably a tablet.

In a second aspect, the present invention relates to a direct compression process for making the pharmaceutical composition according to the first aspect, the process comprising the steps of:
(1) Sieving the compound of formula (1) and at least one excipient and mixing them to obtain a blend;
(2) Tableting the blend of step (1) by compressing it to produce tablet cores.

In a third aspect, the present invention relates to a dry granulation process for making the pharmaceutical composition according to any preceding claims, the process comprising the steps of:
(1) mixing the compound of the formula (1) with at least one excipient in a mixer;
(2) compaction of the mixture of step (1) to form granules, optionally using a roller compactor;
(3) optionally mixing the granules of step (2) with one or more excipients.
(4) compressing the granules of step (2) or the final blend of step (3) to provide a tablet.

A fourth aspect of the invention is the pharmaceutical composition according to the first aspect for use in the treatment of type 2 diabetes.

A fifth aspect of the invention is a blister pack comprising the pharmaceutical composition of the first or fourth aspects wherein said blister pack is preferably an aluminum/PVC blister, an aluminum/aluminum blister, or a PVC/PE/PVDC/aluminum blister.

A sixth aspect of the invention is a cardboard box with a patient information leaflet comprising at least one aluminum/aluminum or aluminum/PVC or PVC/PE/PVDC/aluminum blister pack of at least 4 units of the pharmaceutical composition of the first, fourth or fifth aspects.

### DETAILED DESCRIPTION OF THE INVENTION

The present invention provides immediate release pharmaceutical compositions of compound of the formula (1), empagliflozin.

Empagliflozin is also known by the IUPAC name (2S,3R,4R,5S,6R)-2-[4-Chloro-3-[[4-[(3S)-oxolan-3-yl]oxyphenyl]methyl]phenyl]-6-(hydroxymethyl)oxane-3,4,5-triol. The empirical formula of Empagliflozin is C₂₃H₂₇ClO₇ and the compound has a molecular weight of 450.9 g/mol. The structural formula of Empagliflozin is (1):

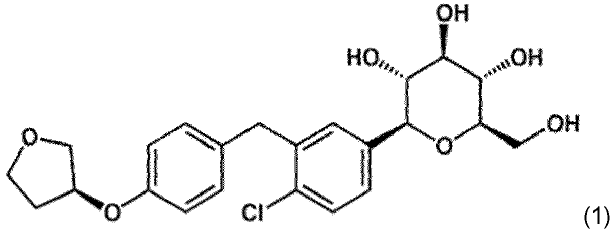

The term empagliflozin and compound of formula (1) are used herein interchangeably.

The aim of the present invention is to provide a pharmaceutical composition with a good dissolution profile that enables a high bioavailability of empagliflozin in a patient, while it avoids or reduces filming and sticking during the manufacturing process of the composition.

Surprisingly, the inventors have found that empagliflozin API with a small particle size, such as micronized grade, presents pharmaceutically acceptable dissolution profiles.

Even more surprisingly, the inventors have found that compositions comprising empagliflozin wherein the PSD is D90 between 1 µm and 10 µm, preferably between 1 µm and 9 µm, more preferably between 4 µm and 9 µm, even more preferably, between 5 µm and 8 µm.

Another aim of the present invention is to provide a pharmaceutical composition that presents a good stability, good friability, good uniformity, good solubility and can be manufactured at industrial scale.

Another aim of the present invention is to provide manufacturing methods for the manufacture of empagliflozin compositions which is easy, effective, competitive in terms of time and costs and environmentally friendly.

Another aim of the present invention is to provide an acceptably sized tablet which can be swallowed easily.

Tablets or capsules which are acceptable for patients are those with dimensions of between 5 mm and 15 mm along the longest axis, more preferably between 5 mm and 10 mm, more preferably between 9 to 6 mm. These dimensions allow the pharmaceutical composition of the first aspect to be swallowed easily.

The term "stable" as used herein refers to a pharmaceutical composition comprising the compound of the formula (1) wherein the content of total impurities is below 5 % (w/w), preferably 3 % (w/w), more preferably 2 % (w/w), and most preferably 1 % (w/w), as determined by liquid chromatography (HPLC) at 225 nm if such a composition is stored for 1 month at 40°C and 75 % relative humidity (RH) or 1 month at 25 °C and 60 % relative humidity (RH).

The term "uniform" as used herein refers to the requirement that the content of the active agent in the tablets or capsules of a pharmaceutical batch has to be homogeneous. Content uniformity is ascertained according to the method specified in European Pharmacopoeia 9th Edition 2.9.6. Uniformity of content of single-dose preparations*.*

The term "active agent" or "active substance" refers to a therapeutically active compound, as well as any prodrugs thereof and pharmaceutically acceptable salts, hydrates and solvates of the compound and the prodrugs.

The term "Dx" as used herein means that x% of the particles in a composition (based on volume) have a diameter of or below a specified d value. Thus, a D90 of 10 µm means that 90% of the particles, by volume, have a diameter of or below 10 µm. As well as using D90 as a measuring reference to determine particle size, D95 is occasionally used for such a purpose. Therefore, a D95 of 10 µm means that 95% of the particles, by volume, have a diameter of or below 10 µm.

The volume mean particle size of the compound of the formula (1) is determined using Malvern Laser Diffraction Mastersizer 3000 with sample feeder Aero (S) and Mastersizer 3000 software, using a general purpose analysis model and enhanced sensitivity. Before measurement the sample was manually homogenized. The hopper was filled with 2-5 g of sample. The vibration rate was adjusted to 25% in order to have obscuration values between 0.5 and 8%.

Three measurements of five seconds' duration were taken over the measuring range of 0.01-3500 µm.

The term "total composition" as used herein means the total composition of components forming the pharmaceutical composition regardless of whether it contains a single phase or multiple phases (e.g. granules). When the pharmaceutical composition comprises granules, the "total composition" will be the sum of the intragranular components and the extragranular components. Because the coating is an optional feature in some embodiments of the invention, the coating is not considered when referring to the "total composition" of a pharmaceutical composition.

In a first aspect, the present invention relates to a pharmaceutical composition comprising at least one active ingredient, wherein said active ingredient is the compound of the formula (1) 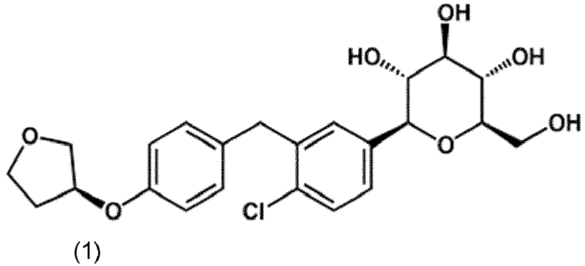 wherein said composition comprises:
at least one diluent,
at least one disintegrant,
at least one lubricant,
wherein the compound of formula (1) has a particle size distribution D90 between 1 and 10 µm,
wherein the composition is prepared preferably by dry methods, and
wherein the composition is preferably a tablet.

In an embodiment of the pharmaceutical composition of the first aspect as herein disclosed, the D90 of the compound of the formula (1) is preferably greater than 4 µm and less than 9 µm as measured by laser diffraction spectroscopy. More preferably, the D90 of the compound of the formula (1) is greater than 5 µm and less than 8 µm as measured by laser diffraction spectroscopy. Even more preferably, the D90 of the compound of the formula (1) is between 6 µm to 7 µm as measured by laser diffraction spectroscopy.

These ranges have been found to improve the flowability of the mixture, and where the pharmaceutical composition of the first aspect is a tablet, to produce harder tablets with lower friability. Surprisingly, it has been found that with this particle size range it is possible to manufacture pharmaceutical compositions by dry methods including direct compression and dry granulation which have a uniform distribution of API and avoid costly wet granulation processes. Surprisingly, it has been possible to produce pharmaceutical compositions with acceptable dissolution profiles using the compound of the formula (1) with a D90 smaller than 10 µm.

In an embodiment of the pharmaceutical composition of the first aspect as herein disclosed, the composition preferably comprises between 3.5 and 25 % by weight of the compound of the formula (1) in respect of the total amount of the pharmaceutical composition of the first aspect. Preferably, it comprises between 5 and 20 % by weight of the compound of the formula (1) in respect of the total amount of the pharmaceutical composition of the first aspect. More preferably, it comprises from about 10 to about 15 % by weight of the compound of the formula (1) in respect of the total amount of the pharmaceutical composition of the first aspect. This allows the manufacture of tablets with an acceptable size across all approved doses from the same formulation. It was also found that this range provided improved dissolution times.

When the pharmaceutical composition of the first aspect as herein disclosed comprises excipients such as a diluent, an acid, a lubricant, a solubiliser or a disintegrant, these excipients are pharmaceutically acceptable. The term "pharmaceutically acceptable" indicates that the substance or composition must be compatible chemically and/or toxicologically, with the other ingredients comprising a formulation, and/or the mammal being treated therewith.

The term "diluent" as used herein refers to pharmaceutically acceptable excipients which are added to the bulk volume of the active agent making up the solid composition. As a result, the size of the solid composition increases, which makes its size suitable for handling. Diluents are convenient when the dose of drug per solid composition is low and the solid composition would otherwise be too small. For the purposes of this application, the terms "filler" and "diluent" are used interchangeably.

The pharmaceutical composition of the first aspect as herein disclosed comprise a diluent. Diluents can be water soluble or water insoluble. Examples of water soluble diluents are lactose, sucrose, mannitol and sorbitol. Examples of water insoluble diluents are starch, powdered cellulose, microcrystalline cellulose and calcium phosphate.

Suitable diluents according to the invention are for example, lactose, in particular lactose monohydrate or lactose anhydrous, cellulose and derivatives, such as powdered cellulose, microcrystalline or silicified microcrystalline cellulose, cellulose acetate, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, sugars and derivatives such as confectioner's sugar, fructose, sucrose, dextrates, dextrin, sorbitol sulfobutylether 11-cyclodextrin, dextrose, polydextrose, trehalose, maltose, maltitol, mannitol, maltodextrin, sorbitol, inulin, xylitol, erythritol, isomalt, kaolin and lactitol. For reasons of patient acceptability the formulation may be free from lactose and microcrystalline cellulose. Some patients cannot digest or metabolise lactose and furthermore, lactose and cellulose derivatives may cause an insulin response.

Preferred diluents for use in the pharmaceutical composition of the first aspect are agglomerated isomalt, lactose monohydrate and microcrystalline cellulose and mixtures thereof. Compositions comprising isomalt or microcrystalline cellulose were found to have improved dissolution and disintegration times, and improved stability. Furthermore, isomalt does not stimulate an insulin response.

In a preferred embodiment, the pharmaceutical composition of the first aspect comprises one or more water insoluble diluents.

One embodiment of the pharmaceutical composition of the first aspect preferably comprises a total amount of diluent or mixture of diluents from about 50 to 95% w/w% of the total composition, preferably 60 to 90%, further preferably 70 to 90%, further preferably 75 to 85%.

As used herein, "disintegrant" means a pharmaceutically acceptable excipient or a mixture of pharmaceutically acceptable excipients added to a formulation to facilitate its breakup or disintegration after administration. In the first aspect, the total amount of disintegrant or disintegrants present in the pharmaceutical composition of the first aspect usually ranges from 1 to 10 % w/w in respect of the total amount of the pharmaceutical composition of the first aspect. In a preferred embodiment of the first aspect, the total amount of disintegrant or disintegrants present in the pharmaceutical composition of the first aspect ranges from 1 to 8% or preferably 2 to 7 % w/w in respect of the total amount of the pharmaceutical composition of the first aspect.

Suitable disintegrants according to the first aspect of the invention are for example powdered cellulose, crospovidone, croscarmellose sodium, docusate sodium, low-substituted hydroxypropyl cellulose, magnesium aluminum silicate, microcrystalline cellulose, polacrilin potassium, silicon dioxide, sodium starch glycolate, starch, particularly pregelatinized starch and cornstarch. Preferred disintegrants according to the first aspect of the invention are colloidal silicon dioxide, croscarmellose sodium and crospovidone.

The composition of the first aspect of the invention preferably comprises intragranular disintegrant, more preferably it comprises both intragranular and extragranular disintegrant.

Suitable intragranular disintegrants are for example powdered cellulose, colloidal silicon dioxide, crospovidone, croscarmellose sodium, low -substituted hydroxypropyl cellulose, magnesium aluminum silicate, microcrystalline cellulose, polacrilin potassium, sodium starch glycolate, starch, particularly pregelatinized starch and cornstarch or a mixture thereof, and is preferably, crospovidone, microcrystalline cellulose, sodium starch glycolate or a mixture thereof.

Suitable extragranular disintegrants are for example powdered cellulose, colloidal silicon dioxide, crospovidone, croscarmellose sodium, low -substituted hydroxypropyl cellulose, magnesium aluminum silicate, microcrystalline cellulose, polacrilin potassium, sodium starch glycolate, starch, particularly pregelatinized starch and cornstarch or a mixture thereof, and is preferably sodium starch glycolate, crospovidone, colloidal silicon dioxide or a mixture thereof.

As a general rule, higher amount of disintegrant enables a faster disintegration, leading to a better dissolution profile and a higher bioavailability. Surprisingly, the inventors of the present invention have found that a lower amount of both intragranular and extragranular disintegrants show the desired dissolution profile.

In an embodiment of the first aspect of the present invention, the disintegrant comprises less than 10% w/w% of the total composition. Preferably, the disintegrant comprises from about 1 to 10% w/w% of the total composition, more preferably from about 1 to 8% w/w%, even more preferably from about 2 to 7% w/w%.

In another embodiment of the first aspect of the present invention, the intragranular disintegrant comprises less than 6% w/w% of the total composition. Preferably, the intragranular disintegrant comprises from about 0.5 to 6% w/w% of the total composition, more preferably from about 1 to 5% w/w%, even more preferably from about 2 to 4.5% w/w%.

In another embodiment of the first aspect of the present invention, the extragranular disintegrant comprises less than 6% w/w% of the total composition. Preferably, the extragranular disintegrant comprises from about 0.5 to 6% w/w% of the total composition, more preferably from about 1 to 5% w/w%, even more preferably from about 2 to 4.5% w/w%.

The term "binder" as used herein is defined as an agent able to bind particles which cannot be bound only by a simple compression force. The binder may be present in the pharmaceutical composition of the first aspect of the invention in the form of a single compound or in the form of a mixture of compounds. Preferably the pharmaceutical composition of the first aspect comprises at least a binder, generally the total amount of binder or mixture of binders is from about 0.5 to 10%, preferably 1-5% and further preferably 2-3% w/w% of the total composition.

Any binder usually employed in pharmaceutical compositions may be used in the pharmaceutical composition of the first aspect. Binders which may be used are for example naturally occurring or partially or totally synthetic polymers selected from acacia, agar, alginic acid, carbomers, carmellose sodium, carrageenan, cellulose acetate phthalate, ceratonia, chitosan, confectioner's sugar, copovidone, povidone, cottonseed oil, dextrate, dextrin, dextrose, polydextrose, maltodextrin, maltose, cellulose and derivatives thereof such as microcrystalline cellulose, methylcellulose, ethylcellulose, hydroxyethyl cellulose, hydroxyethyl methylcellulose, hydroxypropyl celluloses, carboxymethylcelluloses, hypromelloses (cellulose hydroxypropyl methyl ether), starch and derivatives thereof, such as pregelatinized starch, hydroxypropylstarch, corn starch, gelatin, glyceryl behenate, tragacanth, guar gum, hydrogenated vegetable oils, inulin, poloxamer, polycarbophils, polyethylene oxide, polyvinylpyrrolidone, copolymers of N- vinylpyrrolidone and vinyl acetate, polymethacrylates, polyethylene glycols, alginates such as sodium alginate, gelatin, sucrose, sunflower oil, zein as well as derivatives and mixtures thereof. Preferably the pharmaceutical composition of the first aspect is free from polyethylene glycol. Preferred binders include cellulose derivatives such as microcrystalline cellulose, copovidone, crospovidone, lactose anhydrous, or silicified microcrystalline cellulose, pregelatinized starch, low density starch, dibasic calcium phosphate dihydrate, or hydroxypropyl cellulose, hydroxypropylmethyl cellulose or polyvinylpyrrolidone.

In a preferred embodiment, the pharmaceutical composition of the first aspect of the invention comprises a binder selected from povidone, copovidone or crospovidone, preferably copovidone.

As used herein, "lubricant" means a substance that reduces friction between the composition of the present invention and the surfaces of the apparatus used to compact the composition into a compressed form. The pharmaceutical composition of the first aspect comprises at least a lubricant. Preferably, the total amount of lubricant or lubricants present in the pharmaceutical composition of the first aspect ranges from 0.5 to 4% w/w in respect of the total amount of the pharmaceutical composition of the first aspect. More preferably, the total amount of lubricant or lubricants present in the pharmaceutical composition of the first aspect ranges from 1 to 3% w/w in respect of the total amount of the pharmaceutical composition of the first aspect. More preferably, the total amount of lubricant or lubricants present in the pharmaceutical composition of the first aspect ranges from 1 to 2% w/w in respect of the total amount of the pharmaceutical composition of the first aspect.

Preferably, the lubricant of the pharmaceutical composition of the first aspect is selected from magnesium stearate, sodium stearyl fumarate, stearic acid, calcium stearate, glyceryl monostearate, glyceryl palmitostearate, hydrogenated castor oil, hydrogenated vegetable oil, light mineral oil, talc, sodium lauryl sulfate, polyoxyl 40 stearate, polysorbate, polyoxyethylene lauryl ether, sorbitan monooleate, sodium oleate, sodium benzoate, sodium acetate, sodium lauryl sulfate, magnesium lauryl sulfate. More preferably, lubricants are magnesium stearate and sodium stearyl fumarate, particularly preferred is sodium stearyl fumarate. Pharmaceutical compositions comprising magnesium stearate were found to have improved stability. Pharmaceutical compositions comprising sodium stearyl fumarate have been found to have shorter disintegration times and fewer problems such as sticking to the apparatus during manufacture.

As used herein, the term "antiadherent" means any substance that prevents the tablet formulation from sticking to the metal punches and dies used during the manufacturing process.

Any commonly used antiadherent may be used in the pharmaceutical composition of the first aspect, preferably the antiadherent is selected from magnesium stearate, colloidal silicon dioxide, talc and starch. The preferred antiadherents is colloidal silicon dioxide. The antiadherent may be intragranular or extragranular in granulated formulations.

Preferably, the total amount of antiadherent present in the pharmaceutical composition of the first aspect ranges from 0.5 to 2% w/w in respect of the total amount of the pharmaceutical composition of the first aspect.

As used herein, the term "solubiliser" means agents which increase the bioavailability of the API, particularly where poor solubility of the API is a problem.

The pharmaceutical composition of the first aspect may also comprise a solubiliser. Preferably the solubiliser of the pharmaceutical composition of the first aspect is selected from commonly used solubilisers, for example pH modifiers such as citric acid, benzoic acid and tartaric acid; water soluble organic solvents such as polyethylene glycol 300 and 400, ethanol, propylene glycol, glycerin, N-methyl 2-pyrrolidone, dimethyl acetamide; water insoluble organic solvents such as beeswax, D-alpha tocopherol, oleic acid, mono- and di- glycerides; nonionic surfactants such as cremphor, tween 20, sorbitan monooleate, peppermint oil, polysorbate, polysorbate 20 and 80; water insoluble lipids such as peanut oil, corn oil, soybean oil, sesame oil, olive oil, cottonseed oil; cyclodextrins such as alpha, beta and gamma cyclodextrins, sulfobutylether cyclodextrin, hydroxypropyl cyclodextrin; and phospholipids such as glycerol, choline and so on and water soluble polymers such as copovidone or microcrystalline cellulose. Preferred solubilisers are polyvinyl alcohol, microcrystalline cellulose, copovidone, sodium lauryl sulfate and those manufactured under the SEPITRAP^{™} 80 brand or SEPITRAP^{™} 4000 brand. SEPITRAP^{™} 80 comprises between 45 and 65 wt% polysorbate-80 and between 35 and 55 wt% of magnesium aluminometasilicate with respect of the total amount of SEPITRAP^{™} 80. SEPITRAP^{™} 4000 comprises between 55 and 75 wt% of polyoxyl 40 hydrogenated castor oil and between 25 and 45 wt% magnesium aluminometasilicate with respect to the total amount of SEPITRAP^{™} 4000. Surprisingly low quantities of solubiliser were required.

Preferably the pharmaceutical composition of the first aspect comprises at least a solubiliser, generally the total amount of solubiliser or mixture of solubilisers is from about 0 to 10%, preferably 1-5% and further preferably 2-3% w/w% of the total composition.

The pharmaceutical composition of the first aspect is preferably free from polyethylene glycol (PEG). Compositions free from PEG were found to have improved flowability during production.

The preferred pharmaceutical composition of the invention is a tablet composition.

Suitable methods of manufacturing the pharmaceutical composition of the first aspect, preferably a tablet composition, include compression of the pharmaceutical composition of the first aspect in the form of a powder, i.e. direct compression, or compression of the pharmaceutical composition of the first aspect in the form of granules, and if needed with additional excipients, i.e. dry or wet granulation.

Dry methods include direct compression, direct mix and dry granulation. Preferably, dry methods which do not include the addition of any solvent during manufacture of a pharmaceutical composition are employed in the present invention. Dry methods include direct mix, dry granulation and direct compression. Dry methods are preferred for the manufacture of the pharmaceutical composition of the first aspect, preferably a tablet composition, as they were found to produce more stable compositions with better dissolution times. Direct compression and dry granulation are particularly preferred.

In a second aspect, the present invention relates to a direct compression process for making the pharmaceutical composition according to the first aspect, the process comprising the steps of:
(1) Sieving the compound of formula (1) and at least one excipient and mixing them to obtain a blend;
(2) Tableting the blend of step (1) by compressing it to produce tablet cores.

A preferred embodiment of the invention is the direct compression process according to the second aspect, wherein the process comprises the steps of:
(1) Sieving the compound of formula (1) and at least one excipient selected from the diluent, the disintegrant, and the solubiliser and mixing them to obtain a first blend;
(2) Sieving the remaining portions of diluent and disintegrant, optionally an antiadherent and optionally a second solubiliser, adding the obtained mixture to the first blend and mixing to obtain a second blend;
(3) Adding the lubricant to the second blend of step (2) and mixing to obtain a final blend;
(4) Tableting the final blend of step (3) by compressing it on a suitable tablet press to produce tablet cores;
(5) Optionally, film-coating of the tablet cores of step (5) with a film coat.

In a third aspect, the present invention relates to a dry granulation process for making the pharmaceutical composition according to any preceding claims, the process comprising the steps of:
(1) mixing the compound of the formula (1) with at least one excipient in a mixer;
(2) compaction of the mixture of step (1) to form granules, optionally using a roller compactor;
(3) optionally mixing the granules of step (2) with one or more excipients.
(4) compressing the granules of step (2) or the final blend of step (3) to provide a tablet.

A preferred embodiment of the invention is a dry granulation process according to the third aspect of the invention, wherein the process comprises the steps of:
(1) mixing the compound of the formula (1) with the diluent, disintegrant, optionally antiadherent, optionally binder, optionally solubiliser and optionally a first lubricant in a mixer;
(2) compaction of the mixture of step (1) to form granules, optionally using a roller compactor;
(3) optionally mixing the granules of step (2) with a second lubricant in a mixer to obtain the final blend;
(4) compressing the granules of step (3) or the final blend of step (4) to provide a tablet;
(5) optionally film-coating of the product of step (5) with a film coat.

The manufacturing of the pharmaceutical composition of the first aspect by dry methods is more efficient as it demands less steps than a wet method. In addition, dry methods are more environmentally friendly because the use of organic solvents is avoided. Using organic solvents might be needed if manufacturing by wet methods is intended, due to the low solubility of empagliflozin API. Surprisingly, the dissolution profile of empagliflozin formulations according to the present invention prepared by dry methods has been found to be faster than for wet methods.

In a preferred embodiment of the first aspect of the invention, the composition is free of organic solvents, preferably free of any solvent other than water, most preferably essentially free of any solvent.

Alternatively, wet granulation may be used for pharmaceutical compositions of the first aspect.

When the pharmaceutical composition of the present invention is manufactured by dry granulation, the composition comprises granules. The excipients of the composition are then divided in intragranular excipients, extragranular excipients, and optionally a coating. The granules usually comprise the active ingredient (i.e. compound of formula (1)), one or more diluents or binders, one or more disintegrants and optionally a lubricant. The extragranular excipients usually comprise one or more diluents or binders, one or more disintegrants and optionally a lubricant. The coating usually comprises a polymer, an oxide and another excipient, e.g. talc.

A preferred embodiment of the first aspect of the invention is a pharmaceutical composition comprising at least one active ingredient, wherein said active ingredient is the compound of the formula (1) 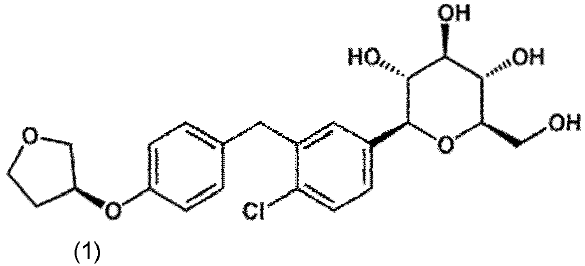 wherein said composition comprises:
at least one diluent,
at least one disintegrant,
at least one lubricant,
wherein the compound of formula (1) has a particle size distribution D90 between 1 and 10 µm,
wherein the composition is prepared preferably by dry methods, and
wherein the composition is preferably a tablet,

And wherein the composition is prepared by dry methods, preferably dry granulation.

Another preferred embodiment of the first aspect of the invention is a pharmaceutical composition comprising at least one active ingredient, wherein said active ingredient is the compound of the formula (1) 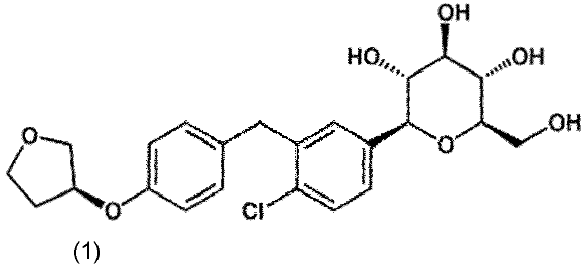 wherein said composition comprises:
at least one diluent,
at least one disintegrant,
at least one lubricant,
wherein the compound of formula (1) has a particle size distribution D90 lower than 10 µm,
wherein the composition is prepared preferably by dry methods, and
wherein the composition is preferably a tablet,
wherein the composition comprises granules, and the composition comprises at least one intragranular disintegrant, preferably at least one intragranular disintegrant and one extragranular disintegrant.

The pharmaceutical composition of the first aspect of the present invention is an immediate release composition, preferably a tablet composition. As used herein, the term "immediate release" refers to the rapid release of the majority of the therapeutic compounds contained in a pharmaceutical composition, allowing said therapeutic compounds to dissolve in the gastrointestinal contents, with no intention of delaying or prolonging the dissolution or absorption of the therapeutic compounds. Preferred conditions for immediate release are the release of at least 80% of the therapeutic compounds within a specified time, typically 45 min or less after oral ingestion, further preferably within 30 minutes and still further preferably within 20 minutes.

In a preferred embodiment of the first aspect of the invention, the pharmaceutical composition in use remains intact in saliva and subsequently disintegrates in the stomach.

The pharmaceutical composition of the first aspect is preferably in the form of tablets. In one embodiment the tablets preferably have a mass of between 50 mg and 300 mg, even more preferably between 75 mg and 250 mg as this range is acceptable to most patients. Preferably they are no longer than 2.5 cm. This ensures patient acceptability.

The pharmaceutical composition of the first aspect is preferably swallowed whole and is preferably not in the form of orally disintegrating tablets. To aid swallowing and prevent dissolution in the mouth, the pharmaceutical composition of the first aspect is preferably coated tablets.

Where the pharmaceutical composition of the first aspect is in the form of tablets, the tablets may be coated with any conventional coating agent to form a film coat. The coating agent may be a polymer based coating agent and preferably comprises a cellulose base polymer such as hypromellose (HPMC) or a polyvinyl alcohol based polymer, such as polyvinyl alcohol (PVA). Preferably the coating agent comprises titanium dioxide and talc. Such coating agents have been found to improve friability and surprisingly, improve stability of the composition. Preferably the coating agent comprises PVA based polymer or a HPMC based polymer. Examples of suitable coating agents are those made under the trade name Opadry^{®}, VlVACOAT^{®} and Methocel^{®}. Preferably the coating comprises no more than 5% of the total mass of the pharmaceutical composition of the first aspect. Further preferably, the coating agent comprises from 1% to 3% of the total mass of the pharmaceutical composition of the first aspect. The coating agent may further comprise colouring or flavouring compounds. The terms "coat", "coating", "film coat" and "film coating" are used interchangeably throughout this specification.

Preferably the composition is film coated and the film coat is a polymer based coating, most preferably a HPMC based coating, for example those produced under the trade name Opadry^{®}, VlVACOAT^{®} and Methocel^{®}.

In a preferred embodiment of the pharmaceutical composition of the first aspect as herein disclosed, the final water content of the pharmaceutical composition of the first aspect is below 7 wt%, preferably from 0 wt% to 5 wt% by weight in respect of the total amount of the pharmaceutical composition of the first aspect. More preferably, the final water content of the pharmaceutical composition of the first aspect is from 0.1 wt% to 2 wt% by weight in respect of the total amount of the pharmaceutical composition of the first aspect. This range has been found to provide improved solubility of the active ingredient, the compound of the formula (1).

In a preferred embodiment of the pharmaceutical composition of the first aspect as herein disclosed, the compound of the formula (1) is in crystalline form, preferably anhydrous crystalline form (II). Preferably, the compound of the formula (1) comprises the crystalline form having an X-ray powder diffraction pattern that comprises peaks at 14.69, 18.84, 19.16, 19.50, 20.36 and 25.21 degrees 2Θ (±0.05° 2Θ). The crystal form is further characterised by a melting point of about 149°C ± 3°C (determined via Differential Scanning Calorimetry using a DSC 821 (Mettler Toledo), as described in WO2006117359 (A1).

Alternatively, the pharmaceutical composition of the first aspect may comprise anhydrous compound of the formula (1). Compositions comprising amorphous compound of the formula (1) were found to be surprisingly stable.

In a preferred embodiment of the pharmaceutical composition of the first aspect as herein disclosed, the compound of the formula (1) is not agglomerated. The term "agglomerated" as used herein refers to the active ingredient, the compound of the formula (1), having an appearance of a crystalline powder with macroscopic agglomerates.

In a preferred embodiment of the pharmaceutical composition of the first aspect as herein disclosed, the pharmaceutical composition of the first aspect comprises between 5 and 50 mg of the compound of the formula (1) per tablet or capsule. Preferably, the pharmaceutical composition of the first aspect comprises 10 mg or 25 mg of the compound of the formula (1) by tablet or capsule.

In a preferred embodiment of the pharmaceutical composition of the first aspect as herein disclosed, are manufactured by dry granulation techniques or dry mix techniques. Preferably, the pharmaceutical composition of the first aspect are manufactured by direct compression or dry granulation.

An embodiment of the present invention relates to a process for the manufacture of a pharmaceutical batch of a tablet of the first or second aspect, wherein the process comprises the following steps:
(i) preparing a test tablet of the compound of the formula (1) according to the first or second aspect;
(ii) checking the stability and dissolution profile of the test tablet of step (i);
   and
(iii) manufacturing a pharmaceutical batch by the same manufacturing process used to prepare the test tablet in step (i) provided that the test tablet is stable and has an immediate release dissolution profile; and
(iv) optionally, packaging the pharmaceutical batch manufactured in step (iii) preferably in blister packs or in bottles.

Another embodiment of the invention is a pharmaceutical batch according to the composition obtained by the first or second aspect validated by a process comprising the following steps:
i) manufacturing the pharmaceutical batch;
ii) checking the uniformity of content of the compound of the formula (1); and
iii) validating the batch only if the content is uniform.

A fourth aspect of the invention is the pharmaceutical composition according to the first aspect for use in the treatment of type 2 diabetes.

A fifth aspect of the invention is a blister pack comprising the pharmaceutical composition of the first or fourth aspects wherein said blister pack is preferably an aluminum/PVC blister, an aluminum/aluminum blister, or a PVC/PE/PVDC/aluminum blister.

A sixth aspect of the invention is a cardboard box with a patient information leaflet comprising at least one aluminum/aluminum or aluminum/PVC or PVC/PE/PVDC/aluminum blister pack of at least 4 units of the pharmaceutical composition of the first, fourth or fifth aspects.

In a preferred embodiment, the pharmaceutical composition of the first aspect is packaged in a blister pack of aluminium/PVC or aluminium/aluminium. In a further embodiment of the pharmaceutical batch as herein disclosed, the pharmaceutical composition of the first aspect as herein disclosed are packaged in aluminium/PVC blisters. Although a blister aluminium/PVC is less protective than the aluminium/aluminium blister, the pharmaceutical composition of the first aspect as herein disclosed shows good stability in aluminum/PVC blisters.

For reasons of patient acceptability, the pharmaceutical composition of the first aspect preferably contains no lactose. Surprisingly, it has been found that compositions prepared by dry methods without lactose have acceptable dissolution profiles.

### EXAMPLES

### Example 1: Tablets comprising empagliflozin manufactured by dry granulation.

| | A | B |
|---|---|---|
| **Intragranular active substance** | **% (w/w)** | |
| Empagliflozin | 12.5 | 12.5 |

| **Intragranular excipients** | | |
|---|---|---|
| MCC | 30.0 | 30.0 |
| Sodium starch glycolate | 4.0 | 4.0 |
| Copovidone | 2.5 | 2.5 |
| Crospovidone | 7.0 | 7.0 |

| **Extragranular excipients** | | |
|---|---|---|
| MCC | 32.5 | 32.5 |
| Sodium starch glycolate | 2.0 | 2.0 |
| Crospovidone | 7.0 | 7.0 |
| Colloidal silicon dioxide | 0.5 | 0.5 |
| Sodium stearyl fumarate | 2.0 | - |
| Magnesium stearate | - | 2.0 |

| **Coating** | | |
|---|---|---|
| Coating agent | 3 | 3 |

Manufacturing process:
1. Screen the empagliflozin, and all the intragranular components through a mesh.
2. Blend the screened components in a bicone.
3. Compact the mix of step 2 using a roller compactor and screen through a mesh.
4. Screen the extragranular cellulose microcrystalline, crospovidone. Sodium starch glycolate and colloidal silicon dioxide through a mesh.
5. Blend the screened components of step 4 with the granules obtained in step 3 in a bicone.
6. Screen the extragranular lubricant through a mesh.
7. Blend the screened lubricant of step 6 with the rest of the components in bicone to obtain the final blend.
10. Tabletting the final blend on a suitable tabletting press.
11. Film coating of the tablet cores.

### Example 2: Tablet comprising empagliflozin manufactured by direct compression.

| **Component** | **% (w/w)** |
|---|---|
| Empagliflozin | 12.5 |
| MCC | 81.0 |
| Crospovidone | 4.0 |
| Colloidal silicon dioxide | 0.5 |
| Magnesium stearate | 2.0 |

Manufacturing process:
1. Screen all components except for magnesium stearate through a mesh.
2. Blend the screened components of step 1 in a high shear mixer.
3. Screen the magnesium stearate through mesh and add it to the mixer with the rest of components.
4. Mix to obtain the final blend.
5. Tabletting of the final blend on a suitable tabletting press.
6. Film coating of the tablet cores.

### Example 3: Tablet comprising empagliflozin manufactured by wet granulation.

| **Component** | **% (w/w)** |
|---|---|
| Empagliflozin | 12.5 |

| **Intragranular excipients** | |
|---|---|
| Lactose monohydrate | 46.0 |
| MCC | 20.0 |
| Sodium starch glycolate | 6.0 |

| **Agglutinant solution** | |
|---|---|
| Copovidone | 4.5 |
| Purified Water | q.s. |

| **Extragranular excipients** | |
|---|---|
| MCC | 10.0 |
| Colloidal silicon dioxide | 0.5 |
| Magnesium stearate | 0.5 |

| Coating | |
|---|---|
| Coating agent | 3 |

Manufacturing process:
1. To obtain the agglutinant solution, copovidone and purified water are mixed in a blender.
2. Screen the empagliflozin and the intragranular excipients through mesh.
3. Blend the screened components of step 2 in a high shear mixer.
4. Load the blend of point 3 into a fluid bed granulator and granulate using the agglutinant solution.
5. Screen the granulate obtained in step 4 through a mesh.
6. Screen the extragranular components colloidal silicon dioxide and microcrystalline cellulose through a mesh.
7. Blend the components of steps 5 and 6 in the mixer.
8. Screen the magnesium stearate through mesh.
9. Mix all components in the mixer to obtain a final blend.
10. Tabletting of the final blend on a suitable tabletting press.
11. Film coating of the tablet cores.

### Example 4: Tablet comprising empagliflozin manufactured by dry granulation without binder

| **Component** | **% (w/w)** |
|---|---|
| Empagliflozin | 12.5 |

| **Intragranular excipients** | |
|---|---|
| MCC | 30.0 |
| Sodium starch glycolate | 4.0 |

| **Extragranular excipients** | |
|---|---|
| MCC | 49.0 |
| Sodium starch glycolate | 2.0 |
| Colloidal silicon dioxide | 0.5 |
| Magnesium stearate | 2.0 |

| **Coating** | |
|---|---|
| Coating agent | 3 |

### Example 5: Dissolution profiles of Tablets manufactured in Examples 1-4

Dissolution profiles of tablets manufactured according to Examples 1-4 with a PSD below 10 microns showed satisfactory dissolution profiles, all showing a dissolution rate at 15 minutes equal or greater to 85% of dissolved empagliflozin.

The solubility of the formulation of Example 1 was assessed using a USP paddle apparatus with 900 mL of pH 6.8 phosphate buffer. The following table shows an average of 6 measurements (6 vessels).

| Example | PSD (D90) | % dissolved (15 min) | % dissolved (30 min) | % dissolved (60 min) |
|---|---|---|---|---|
| 1A | 4 µm | MT 85 | MT 85 | MT 85 |
| 1A | 17 µm | LT 85 | MT 85 | MT 85 |
| 1B | 8 µm | MT 85 | MT 85 | MT 85 |
| 1B | 30 µm | LT 85 | LT 85 | LT 85 |

| | | | | |
|---|---|---|---|---|
| MT: More than LT: Less than | | | | |

### Example 6 - Tablets comprising empagliflozin manufactured by granulation

| **Component** | **mg/tablet** |
|---|---|
| Empagliflozin | 12.5 |
| Metformin HCl | 1000 |

| **Intragranular excipients** | |
|---|---|
| | |
| Sodium Lauryl Sulphate | 35.0 |
| MCC | 52.0 |
| Sodium starch glycolate | 60.0 |

| **Agglutinant solution** | |
|---|---|
| HPMC | 4.5 |
| Purified Water | q.s. |

| **Extragranular excipients** | |
|---|---|
| Sodium starch glycolate | 30.0 |
| Colloidal silicon dioxide | 6.0 |
| Magnesium stearate | 9.0 |

| Coating | |
|---|---|
| Coating agent | 3 |

Manufacturing process:
1. To obtain the agglutinant solution, HPMC and purified water are mixed in a blender.
2. Screen the metformin and the Sodium starch glycolate and MCC excipients through mesh.
3. Blend the screened components of step 2 in a fluid bed.
4. Disperse empagliflozin in water and sodium lauryl sulphate.
5. Load the blend of point 3 into a fluid bed granulator and granulate using the agglutinant solution of HPMC and water, then spray the dispersion of empagliflozin and sodium lauryl sulphate.
6. Screen the granulate obtained in step 5 through a mesh.
7. Screen the extragranular components colloidal silicon dioxide and sodium starch glycolate through a mesh.
8. Blend the components of steps 6 and 7 in the mixer.
9. Screen the magnesium stearate through mesh.
10. Mix all components in the mixer to obtain a final blend.
11. Tabletting of the final blend on a suitable tabletting press.
12. Film coating of the tablet cores.

### Example 7: Tablets comprising empagliflozin manufactured by granulation without binder.

| | **A** | **B** |
|---|---|---|
| **Intragranular active substance** | **% (w/w)** | |
| Empagliflozin | 12.5 | 12.5 |

| **Intragranular excipients** | | |
|---|---|---|
| Sodium Lauryl Sulphate | 3.5 | 3.5 |
| MCC | 30.0 | 30.0 |
| Sodium starch glycolate | 4.0 | 4.0 |
| Copovidone | 2.5 | 2.5 |
| Crospovidone | 7.0 | 7.0 |

| **Extragranular excipients** | | |
|---|---|---|
| MCC | 32.5 | 32.5 |
| Sodium starch glycolate | 2.0 | 2.0 |
| Crospovidone | 7.0 | 7.0 |
| Colloidal silicon dioxide | 0.5 | 0.5 |
| Sodium stearyl fumarate | 2.0 | - |
| Magnesium stearate | - | 2.0 |

| **Coating** | | |
|---|---|---|
| Coating agent | 3 | 3 |

Manufacturing process:
1. Disperse empagliflozin in water and sodium lauryl sulphate.
2. Screen the rest of intragranular excipients and blend using a bicone.
3. Load the blend of point 2 into a fluid bed granulator and spray the dispersion of empagliflozin and sodium lauryl sulphate.
4. Screen the granulate obtained in step 3 through a mesh.
5. Screen the extragranular cellulose microcrystalline, crospovidone. Sodium starch glycolate and colloidal silicon dioxide through a mesh.
6. Blend the screened components of step 5 with the mixture obtained in step 3 in a bicone.
7. Blend the screened lubricant of step 6 with the rest of the components in bicone to obtain the final blend.
8. Tabletting the final blend on a suitable tabletting press.
9. Film coating of the tablet cores.

### Example 8: Dissolution profiles of Tablets manufactured in Examples 6 and 7

Dissolution profiles of tablets manufactured according to Examples 6 and 7 with a PSD below 10 microns and PSD above 100 microns showed satisfactory dissolution profiles, all showing a dissolution rate at 15 minutes equal or greater to 85% of dissolved empagliflozin.

The solubility of the formulation of Examples 6 and 7 was assessed using a USP paddle apparatus with 900 mL of pH 6.8 phosphate buffer. The following table shows an average of 6 measurements (6 vessels).

| Example | PSD (D90) | % dissolved (15 min) | % dissolved (30 min) | % dissolved (60 min) |
|---|---|---|---|---|
| 6A | 4 µm | MT 85 | MT 85 | MT 85 |
| 6B | 130 µm | MT 85 | MT 85 | MT 85 |
| 7A | 4 µm | MT 85 | MT 85 | MT 85 |
| 7B | 130 µm | MT 85 | MT 85 | MT 85 |

| | | | | |
|---|---|---|---|---|
| MT: More than LT: Less than | | | | |

### Particle Size Distribution Measurement by Laser Diffraction

Particle Size Distribution is performed for example via light scattering or laser diffraction technique. To determine the particle size the powder is fed into a laser diffraction spectrometer for example by means of a dispersing unit. The test method is described below in detail:

| | |
|---|---|
| Equipment: | Mastersizer3000 serial number MAL1080693 |
| Accessory Name: | Manual Accessory |
| Sample Dispersing Unit: | HydroSM |
| Dispersant Medium: | Water with 0.1% Tween80 |
| Stirrer Speed: | 2000 rpm |
| Sonication: | No |
| Optical Model: | Mie Theory |
| Material Absorption Index: | 0.01 |
| Material Refraction Index: | 1.52 |
| Number of measurements: | 2 replicates |

Each replicate consists of the average of three consecutive measurements which were stable and reproducible. The adjust with the corresponding optical model was satisfactory.

### Dissolution Test

The standard dissolution test is described in USP31-NF26 S2, chapter 711 (dissolution). For the experiments present in this patent, the paddle method with an agitation speed of 50 rpm was used. The medium consisted of phosphate buffer with a pH of 6.8 and at a temperature of 37°C. The volume of medium per vessel was 900 ml. The tests are usually run for as long as 60 minutes. The samples are analysed by HPLC.

Further aspects and embodiments of the present invention can be found in the following numbered clauses:
1. A pharmaceutical composition comprising at least one active ingredient, wherein said active ingredient is the compound of the formula (1) 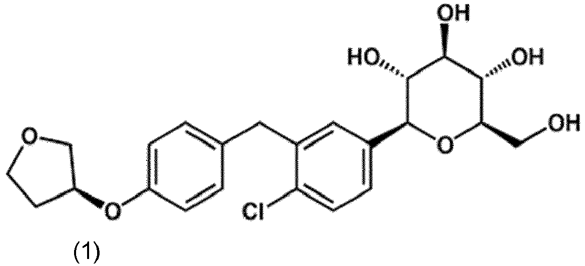 wherein said composition comprises:
   at least one diluent,
   at least one disintegrant,
   at least one lubricant,
   optionally a binder,
   wherein the compound of formula (1) has a particle size distribution D90 between 1 and
   10 µm,
   wherein the composition is prepared preferably by dry methods, and
   wherein the composition is preferably a tablet.
2. The composition according to clause 1 wherein the dry methods comprise direct mix, direct compression or dry granulation, more preferably, dry granulation.
3. The composition according to any preceding clause wherein the compound of formula (1) has a particle size distribution D90 between 1 µm and 9 µm, preferably between 4 µm and 9 µm, more preferably, between 5 µm and 8 µm.
4. The composition according to any preceding clause wherein the compound of formula (1) in the composition is in amorphous form or in crystalline form.
5. The composition according to any of the preceding clauses wherein when the compound of formula (1) is in crystalline form, is present as a monohydrate.
6. The composition according to any of the preceding clauses wherein the compound of formula (1) is anhydrous.
7. The composition according to any of the preceding clauses wherein when the compound of formula (1) is in crystalline form, has an X-ray powder diffraction pattern that comprises peaks at 14.69, 17.95, 18.84, 19.16, 19.50, 20.36, 23.44, 25.21 degrees 2Θ (±0.05° 2Θ) obtained using CuKα radiation.
8. The composition according to any preceding clauses wherein the diluent is selected from a carbohydrate or a carbohydrate derivative, lactose, preferably lactose monohydrate, cellulose and derivatives, such as powdered cellulose, microcrystalline or silicified microcrystalline cellulose, cellulose acetate, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, sugars and derivatives such as confectioner's sugar, fructose, sucrose, dextrates, dextrin, sorbitol sulfobutylether 11-cyclodextrin, dextrose, polydextrose, trehalose, maltose, maltitol, mannitol, maltodextrin, sorbitol, inulin, xylitol, erythritol, isomalt, lactose monohydrate, kaolin and lactitol, and is preferably agglomerated isomalt, lactose or microcrystalline cellulose.
9. The composition according to any preceding clause wherein the disintegrant is selected from powdered cellulose, crospovidone, croscarmellose sodium, low-substituted hydroxypropyl cellulose, magnesium aluminum silicate, microcrystalline cellulose, polacrilin potassium, sodium starch glycolate, starch, particularly pregelatinized starch and cornstarch, and is preferably sodium starch glycolate, crospovidone or a mixture thereof.
10. The composition according to any of the preceding clauses wherein the composition comprises intragranular disintegrant, wherein the composition preferably comprises both intragranular and extragranular disintegrant.
11. The composition according to any of the preceding clauses, wherein the intragranular and extragranular disintegrants are selected from powdered cellulose, crospovidone, croscarmellose sodium, low -substituted hydroxypropyl cellulose, magnesium aluminum silicate, microcrystalline cellulose, polacrilin potassium, sodium starch glycolate, starch, particularly pregelatinized starch and cornstarch or a mixture thereof, and is preferably, crospovidone, sodium starch glycolate or a mixture thereof.
12. The composition according to any preceding clauses wherein the lubricant is selected from magnesium stearate, sodium stearyl fumarate, stearic acid, calcium stearate, glyceryl monostearate, glyceryl palmitostearate, hydrogenated castor oil, hydrogenated vegetable oil, light mineral oil, talc, sodium lauryl sulfate, polyoxyl 40 stearate, polysorbate, polyoxyethylene lauryl ether, sorbitan monooleate, sodium oleate, sodium benzoate, sodium acetate, sodium lauryl sulfate, magnesium lauryl sulfate or a mixture thereof and is preferably sodium stearyl fumarate, magnesium stearate or a mixture thereof.
13. The composition according to any preceding clauses wherein the composition further comprises at least a binder and optionally wherein the binder is selected from microcrystalline cellulose, copovidone, crospovidone, lactose anhydrous, or silicified microcrystalline cellulose, pregelatinised starch, low density starch, dibasic calcium phosphate dihydrate, or hydroxypropyl cellulose, hydroxypropylmethyl cellulose or polyvinylpyrrolidone, preferably copovidone.
14. The composition according to any preceding clauses further comprising at least an antiadherent, optionally wherein the antiadherent is selected from magnesium stearate, colloidal silicon dioxide, talc and starch, and preferably wherein the antiadherent is colloidal silicon dioxide.
15. The composition according to any preceding clause further comprising at least a solubiliser, optionally wherein the solubiliser is a polymer based on vinyl- pyrrolidone such as a copolymer of vinyl- pyrrolidone and vinyl acetate, preferably copovidone, or sodium lauryl sulfate or a mixture of polysorbate-80 and magnesium aluminometasilicate or polyoxyl 40 hydrogenated castor oil and magnesium aluminometasilicate, or alternatively the solubilizer is microcrystalline cellulose or a mixture of solubilisers.
16. The composition according to any preceding clause wherein the composition comprises the compound of formula (1) in an amount from about 3.5 to 25% w/w% of the total composition, preferably from about 5 to 20% w/w%, preferably from about 10 to 15% w/w% .
17. The composition according to any preceding clause wherein the total amount of diluent or mixture of diluents is from about 50 to 95% w/w%, preferably from 60 to 85% w/w%, more preferably from 70 to 80% w/w% of the total composition.
18. The composition according to any preceding clause wherein the lubricant comprises from 0.5 to 4 w/w% of the total composition, preferably from about 1 to 3% w/w% , further preferably 1 to 2% w/w% .
19. The composition according to any preceding clause wherein the disintegrant comprises from about 5 to 30% w/w% of the total composition, preferably from about 10 to 25% w/w%, further preferably from about 15 to 20% w/w%.
20. The composition according to any preceding clause wherein when the composition comprises the compound of formula (1) in an amount from about 3.5 to 25% w/w%, the total amount of diluent or mixture of diluents is from about 50 to 95% w/w% of the total composition, preferably 60 to 90% w/w%, further preferably 70 to 90% w/w%, further preferably 75 to 85% w/w%;
   or when the composition comprises the compound of formula (1) in an amount above 25% w/w%, the total amount of diluent or mixture of diluents is from about 45 to 75% w/w% of the total composition, preferably 50 to 70%, further preferably 60 to 70%.
21. The composition according to any preceding clause wherein the total amount of lubricant is from about 0 to 2% w/w%, preferably 0.5 to 2% w/w%, further preferably from about 0.5 to 1% w/w% of the total composition.
22. The composition according to any preceding clause wherein the total amount of solubiliser is from about 0 to 10% w/w%, preferably about 3 to 8 w/w% of the total composition.
23. The composition according to any preceding clause which is free of lactose.
24. The composition according to any preceding clause which is free of hydroxypropyl cellulose.
25. The composition according to any preceding clause further comprising a coating.
26. The composition according to the preceding claim, wherein the coating comprises a cellulose based polymer, preferably hypromellose, or a polyvinyl alcohol based polymer, preferably polyvinyl alcohol, titanium dioxide and talc.
27. The composition according to any of the two preceding clauses, wherein the coating comprises no more than 5% of the total mass of the pharmaceutical composition, preferably from 1% to 3% of the total mass of the pharmaceutical composition.
28. The composition according to any preceding clause, wherein the composition comprises microcrystalline cellulose and the compound of formula (1) has a D90 lower than 10 µm.
29. The composition according to any preceding clause further comprising metformin or metformin hydrochloride.
30. The composition according to any preceding clause further comprising a DPP-IV inhibitor, preferably linagliptin.
31. A direct compression process for making the pharmaceutical composition according to any preceding clause, the process comprising the steps of:
   (1) Sieving the compound of formula (1) and at least one excipient and mixing them to obtain a blend;
   (2) Tableting the blend of step (1) by compressing it to produce tablet cores.
32. The direct compression process according to the preceding clause, wherein the process comprises the steps of:
   (1) Sieving the compound of formula (1) and at least one excipient selected from the diluent, the disintegrant, and the solubiliser and mixing them to obtain a first blend;
   (2) Sieving the remaining portions of diluent and disintegrant, optionally an antiadherent and optionally a second solubiliser, adding the obtained mixture to the first blend and mixing to obtain a second blend;
   (3) Adding the lubricant to the second blend of step (2) and mixing to obtain a final blend;
   (4) Tableting the final blend of step (3) by compressing it on a suitable tablet press to produce tablet cores;
   (5) Optionally, film-coating of the tablet cores of step (5) with a film coat.
33. A dry granulation process for making the pharmaceutical composition according to any of clauses 1-30, the process comprising the steps of:
   (1) mixing the compound of the formula (1) with at least one excipient in a mixer;
   (2) compaction of the mixture of step (1) to form granules, optionally using a roller compactor;
   (3) optionally mixing the granules of step (2) with one or more excipients.
   (4) compressing the granules of step (2) or the final blend of step (3) to provide a tablet.
34. The dry granulation process according to the preceding clause, wherein the process comprises the steps of:
   (1) mixing the compound of the formula (1), with the diluent, disintegrant, optionally antiadherent, optionally binder, optionally solubiliser and optionally a first lubricant in a mixer;
   (2) compaction of the mixture of step (1) to form granules, optionally using a roller compactor;
   (3) optionally mixing the granules of step (2) with a second lubricant in a mixer to obtain the final blend;
   (4) compressing the granules of step (3) or the final blend of step (4) to provide a tablet;
   (5) optionally film-coating of the product of step (5) with a film coat.
35. The pharmaceutical composition according to any of clauses 1-30 for use in the treatment of type 2 diabetes.
36. The pharmaceutical composition of any of clauses 1 to 30 and 35, wherein the composition is a tablet.
37. The pharmaceutical composition according to any preceding clause which is not an orally disintegrating tablet.
38. The pharmaceutical composition according to any of the clauses 1 to 30 wherein the binder is copovidone.
39. The pharmaceutical composition according to the preceding clause wherein the the disintegrant or disintegrants mixture comprises crospovidone and/or sodium starch glycolate.
40. The pharmaceutical composition according to any of the clauses 1 to 30 or 35 to 39 with the proviso that the dissolution rate of empagliflozin at 15 minutes is equal or greater than 85% when measured with a USP paddle apparatus in a 900 mL phosphate buffer medium at pH 6.8, agitation speed of 50 rpm and a temperature of 37°C.
41. A pharmaceutical composition comprising at least one active ingredient, wherein said active ingredient is the compound of the formula (1) 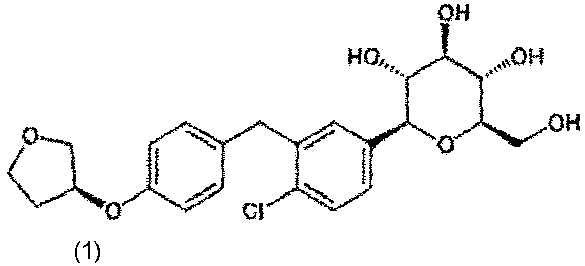 wherein said composition comprises:
   at least one diluent,
   at least one disintegrant,
   at least one lubricant,
   optionally a binder,
   wherein the compound of formula (1) has a particle size distribution D90 between 101 and 130 µm,
   wherein the composition is prepared preferably by granulation methods,
   wherein the composition is prepared by dispersing empagliflozin in a water solution which is free from a binder and
   wherein the composition is a tablet.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Tablets produced according to the above clauses have acceptable disintegration times and friability. The target disintegration time is 3 minutes or less and the target friability of <1%.

Unless otherwise indicated, all the analysis methods are carried out according to the European Pharmacopoeia 7th edition.

## Claims

1. A pharmaceutical composition comprising at least one active ingredient, wherein said active ingredient is a crystalline compound of the formula (1) 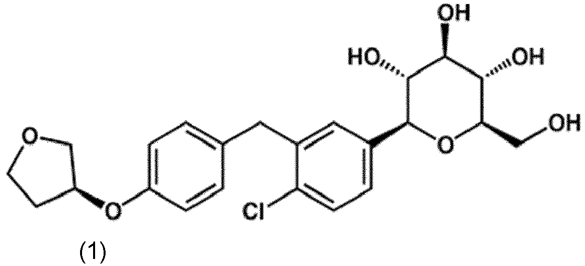 wherein said composition comprises:
at least one diluent,
at least one disintegrant,
at least one lubricant,
optionally a binder,
wherein the compound of formula (1) has a particle size distribution D90 is between 1 and 10 µm,
wherein the composition is prepared by dry methods, and
wherein the composition is a tablet.

2. The pharmaceutical composition according to claim 1 wherein the dry methods comprise direct mix, direct compression or dry granulation, more preferably, dry granulation.

3. The pharmaceutical composition according to any preceding claim wherein the crystalline compound of formula (1) has a particle size distribution D90 between 1 µm and 9 µm, preferably between 4 µm and 9 µm, more preferably, between 5 µm and 8 µm.

4. The pharmaceutical composition according to any of the preceding claims wherein when the compound of formula (1) is present as a monohydrate.

5. The pharmaceutical composition according to any of the preceding claims wherein the compound of formula (1) is anhydrous.

6. The pharmaceutical composition according to any of the preceding claims wherein when the compound of formula (1) is in crystalline form, has an X-ray powder diffraction pattern that comprises peaks at 14.69, 17.95, 18.84, 19.16, 19.50, 20.36, 23.44, 25.21 degrees 2Θ (±0.05° 2Θ) obtained using CuKα radiation.

7. The pharmaceutical composition according to any preceding claim wherein the diluent is selected from a carbohydrate or a carbohydrate derivative, lactose, preferably lactose monohydrate, cellulose and derivatives, such as powdered cellulose, microcrystalline or silicified microcrystalline cellulose, cellulose acetate, starches and derivatives such as pregelatinized starch, corn starch, wheat starch, rice starch, potato starch, sterilizable maize, sodium chloride, calcium carbonate, calcium phosphate, particularly dibasic calcium phosphate, calcium sulphate, dicalcium or tricalcium phosphate, magnesium carbonate, magnesium oxide, sugars and derivatives such as confectioner's sugar, fructose, sucrose, dextrates, dextrin, sorbitol sulfobutylether 11-cyclodextrin, dextrose, polydextrose, trehalose, maltose, maltitol, mannitol, maltodextrin, sorbitol, inulin, xylitol, erythritol, isomalt, lactose monohydrate, kaolin and lactitol, and is preferably agglomerated isomalt, lactose or microcrystalline cellulose.

8. The pharmaceutical composition according to any preceding claim wherein the disintegrant is selected from powdered cellulose, crospovidone, croscarmellose sodium, low-substituted hydroxypropyl cellulose, magnesium aluminum silicate, microcrystalline cellulose, polacrilin potassium, sodium starch glycolate, starch, particularly pregelatinized starch and cornstarch, and is preferably sodium starch glycolate, crospovidone, or a mixture thereof.

9. The pharmaceutical composition according to any of the preceding claims wherein the composition comprises intragranular disintegrant, wherein the composition preferably comprises both intragranular and extragranular disintegrant.

10. The pharmaceutical composition according to any of the preceding claims, wherein the intragranular and extragranular disintegrants are selected from powdered cellulose, crospovidone, croscarmellose sodium, low -substituted hydroxypropyl cellulose, magnesium aluminum silicate, microcrystalline cellulose, polacrilin potassium, sodium starch glycolate, starch, particularly pregelatinized starch and cornstarch or a mixture thereof, and is preferably, crospovidone, sodium starch glycolate or a mixture thereof.

11. The pharmaceutical composition according to any preceding claim wherein the lubricant is selected from magnesium stearate, sodium stearyl fumarate, stearic acid, calcium stearate, glyceryl monostearate, glyceryl palmitostearate, hydrogenated castor oil, hydrogenated vegetable oil, light mineral oil, talc, sodium lauryl sulfate, polyoxyl 40 stearate, polysorbate, polyoxyethylene lauryl ether, sorbitan monooleate, sodium oleate, sodium benzoate, sodium acetate, sodium lauryl sulfate, magnesium lauryl sulfate or a mixture thereof and is preferably sodium stearyl fumarate, magnesium stearate or a mixture thereof.

12. The pharmaceutical composition according to any preceding claim wherein the composition further comprises at least a binder and optionally wherein the binder is selected from microcrystalline cellulose, copovidone, crospovidone, lactose anhydrous, or silicified microcrystalline cellulose, pregelatinised starch, low density starch, dibasic calcium phosphate dihydrate, or hydroxypropyl cellulose, hydroxypropylmethyl cellulose or polyvinylpyrrolidone, preferably copovidone.

13. The pharmaceutical composition according to any of the preceding claims wherein the composition comprises a greater amount of disintegrant than binder, more preferably, the amount of disintegrant in the composition (% w/w) is at least 5 times greater the amount of binder.

14. The pharmaceutical composition according to any preceding claim further comprising at least an antiadherent, optionally wherein the antiadherent is selected from magnesium stearate, colloidal silicon dioxide, talc and starch, and preferably wherein the antiadherent is colloidal silicon dioxide.

15. The pharmaceutical composition according to any preceding claim further comprising at least a solubiliser, optionally wherein the solubiliser is a polymer based on vinylpyrrolidone such as a copolymer of vinyl- pyrrolidone and vinyl acetate, preferably copovidone, or sodium lauryl sulfate or a mixture of polysorbate-80 and magnesium aluminometasilicate or polyoxyl 40 hydrogenated castor oil and magnesium aluminometasilicate, or alternatively the solubilizer is microcrystalline cellulose or a mixture of solubilisers.
